Europäisches Patentamt

European Patent Office

Office européen des brevets

(11) Publication number: **0 091 760**
**B1**

(12) # EUROPEAN PATENT SPECIFICATION

(45) Date of publication of patent specification: **02.07.86**

(51) Int. Cl.⁴: **G 01 N 33/564, C 07 K 15/14**

(21) Application number: **83301845.0**

(22) Date of filing: **31.03.83**

(54) **Anti immune complex antibody and preparation thereof.**

(30) Priority: **09.04.82 JP 58274/82**
**29.11.82 JP 207658/82**

(43) Date of publication of application:
**19.10.83 Bulletin 83/42**

(45) Publication of the grant of the patent:
**02.07.86 Bulletin 86/27**

(84) Designated Contracting States:
**DE FR GB NL**

(56) References cited:
FR-A-2 362 929
FR-A-2 374 330
FR-A-2 480 782
GB-A-2 001 171
US-A-4 283 383

CHEMICAL ABSTRACTS, vol. 88, 1978, page 437, no. 168279p, Columbus, Ohio, USA, K. KANO et al.: "Detection of circulating immune complexes by the inhibition of anti-antibody"

CHEMICAL ABSTRACTS, vol. 69, no. 21, 1968, abstract no. 85012c, Columbus, Ohio, USA, H.L. SPIEGELBERG et al.: "The production of antisera to human gammaG subclasses in rabbits using immunological unresponsiveness"

(73) Proprietor: **FUJIREBIO KABUSHIKI KAISHA also trading as FUJIREBIO INC.**
**6-7, Shimoochiai 4-chome Shinjuku-ku Tokyo 161 (JP)**

(72) Inventor: **Soma, Kazunori**
**130, 22-10, Kitashinjuku 3-chome Shinjuku-ku Tokyo (JP)**
Inventor: **Kasahara, Yasushi**
**310, 4-4, Nagayama 3-chome Tama-shi Tokyo (JP)**

(74) Representative: **Silverman, Warren et al HASELTINE LAKE & CO. Hazlitt House 28 Southampton Buildings Chancery Lane London WC2A 1AT (GB)**

(56) References cited:

BIOLOGICAL ABSTRACTS, vol. 71, no. 7, 1981, abstract no. 45557, H. NASU et al.: "Naturally occurring human antibodies to F(ab')2 portion of immunoglobulin G"

CHEMICAL ABSTRACTS, vol. 69, 1968, page 4716, no. 50531u, Columbus, Ohio, USA, C.I. LUKS et al.: "Aggregation of an immunoglobulin fragment by sulfhydryl oxidation"

Courier Press, Leamington Spa, England.

(56) References cited:

CHEMICAL ABSTRACTS, vol. 90, 1979, page 448, no. 20592f, Columbus, Ohio, USA, L. GRANGEOT-KEROS et al.: "Detection of immune complexes: a simple assay based on characterization of the in vivo bound Clq (PEG-Clq immunodiffusion test)"

CHEMICAL ABSTRACTS, vol. 96, 1982, page 471, no. 32997d, Columbus, Ohio, USA, M.P. DAVEY: "Development and evaluation of new tests for circulating immune complexes: enzyme-linked solid phase mRF, Clq, and F(ab')2 binding assays"

"CLINICAL ASPECTS OF IMMUNOLOGY", 1963, Chapter XXV, pages 593-610, Blackwell Scientific Publications, Oxford, GB., L.E. GLYNN: "Rheumatoid arthritis and the rheumatoid factor"

CHEMICAL ABSTRACTS, vol. 95, 1981, page 495, no. 113087p, Columbus, Ohio, USA, T. NISHIMAKI et al.: "Various procedures for the detection of circulating immune complexes"

**Description**

This invention relates to a novel antibody capable of detecting a wide variety of immune complexes and to a method for its preparation.

An immune complex is the combination product of an antigen, an antibody and a complement. When such an immune complex is formed in the human body, the immune complex is usually made harmless by leucocytes or macrophages. However, when a large quantity of antigen exists in the human body, or when an antigen whose antibody is difficult to form exists in the human body, the amount of immune complex increases, and this gives rise to various diseases such as acute glomerulonephritis, angitis, chronic urticaria, and thromboxytopenia.

With a view to preventing such diseases, there have been developed various methods for measuring such an immune complex. In one such method, the reaction of the complement or rheumatoid factor with the immune complex is measured and in another the reaction of a Fc receptor with the immune complex is utilised. Other methods are of a physicochemical nature, such as gel filtration, sucrose density gradient centrifugation and precipitation with polyethyleneglycol. However, both the method using the complement or rheumatoid factor and the method using a Fc receptor have the fatal defect that these methods cannot distinguish between aggregated IgG and the immune complex. The physiocochemical methods are complicated, and of insufficient specificity.

Recently, a new method has been reported which uses an anti-antibody which recognizes structural change in the Fab fragment caused by the combination of antigen and antibody (K. Kano et al., Clinical Immunology and Immunopathology, vol. 9, pp 425—435 (1978)). This method utilizes the inhibition of the immune complex in a sample blood serum which inhibits agglutination between anti-antibody having 3 agglutinating units and anti-D antibody sensitized blood cell. This method is easy to carry out and specifically detects an immune complex and it does not detect the aggregated IgG. However, this anti-antibody is not easily obtained.

It is an object of this invention to provide an antibody which is readily available from a variety of convenient source materials and which is capable of detecting a variety of immune complexes.

According to this invention, there is provided an anti-immune complex antibody which is a glycoprotein having a molecular weight of from 150,000 to 180,000 when determined by the gel filtration method, which antibody reacts with an immune complex in blood serum of patient subject to systemic lupus erythematosus and with an immune complex in blood serum of a patient subject to rheumatoid arthritis, but does not react with aggregated IgG, has a sedimentation coefficient of from 6.5 to 7.1 determined by the Meniscus depletion method, has a saccharide content of from 2% to 4.5% by weight as determined by the Phenol-Sulphuric acid method and has an isoelectric point of from 5.8 to 8.7.

More specifically, it has now been found that when a complex of an antigen and $F(ab')_2$ fragment of human antibody of this antigen is injected into a warm-blooded animal to form a novel antibody into the body of the animal, this novel antibody specifically detects the immune complex of that antigen as well as various other immune complexes. An antibody of this type can also be produced by injecting an aggregate of $F(ab')_2$ fragment of human immunoglobulin as antigen in a warm-blooded animal such as rabbit. It has also been found that the antibody can be produced as monoclonal antibody from a hybridoma derived from a warm blooded animal to which such a complex or such an aggregate has been injected.

The properties of one anti-immune complex antibody of the invention, that obtained in Example 1, are as follows:

(1) Reactions with immune complexes and aggregated human IgG,

The antibody reacts with the immune complexes in the blood sera of patients suffering from systemic lupus erythematosus and chronic articular rheumatism and with the immune complex of inactivated tetanus toxoid and anti-tetanus toxoid human IgG. On the other hand, the antibody does not react with aggregated IgG obtained by heating or with IgG obtained from its immune complex. The above measurements were carried out according to the ELISA method which is described in Example 4 hereinafter.

The reactivity of the antibody of the invention with respect to the complex of tetanus toxoid and anti-tetanus toxoid human IgG, and with respect to tetanus toxoid, anti-tetanus toxoid human IgG, human IgG, aggregated human IgG, human $F(ab')_2$ fragment, and aggregated human $F(ab')_2$ fragment were measured by the Ouchterlony method, which is an immunodiffusion method. Precipitation lines appeared between the antibody and the aforementioned complex and between the antibody and the aggregated human $F(ab')_2$ fragment, but did not appear between the antibody and the other indicated substances.

(2) Molecular weight

Antibodies obtained from the blood sera of rabbit and guinea pig:

150,000—160,000 (gel filtration)

150,000 (SDS polyacrylamide electrophoresis)

Antibody obtained from the blood serum of chicken:

160,000—180,000 (gel filtration)

(3) Sedimentation coefficient

Antibodies obtained from the blood sera of rabbit and guinea pig:

6.5 (Meniscus depletion method)

Antibody obtained from the blood serum of chicken:

7.1 (Meniscus depletion method)

(4) Saccharide content
Antibodies obtained from the blood sera of rabbit and guinea pig:

2—4% (Phenol-Sulphuric acid method)

Antibody obtained from the blood serum of chicken:

3.5—4.5% (Phenol-Sulphuric acid method)

(5) Isoelectric point
Antibody obtained from the blood sera of rabbit and guinea pig:

5.8—8.7

The foregoing complexes of an antigen and the F(ab')$_2$ fragment of human immunoglobulin are novel materials, never previously having being proposed for injection into any living body. When such an antibody is compared with the anti-antibody employed in the Kano method, since the antibody of this invention is obtained from a warm-blooded animal other than a human being, whereas the anti-antibody of the Kano method was essentially obtained from a human body, this antibody is fundamentally different from the anti-antibody.

An antibody of this invention may be prepared by using the complex of an antigen and F(ab')$_2$ fragment of human antibody of this antigen or by using the aggregate of F(ab')$_2$ fragment of human immunoglobulin as its immunogens.

When the antibody of this invention is prepared by using the complex of an antigen and F(ab')$_2$ fragment of human antibody to this antigen, it does not matter, in principle, what antigen is used. However, it is preferred that the antigen is water-soluble. Among antigens which can be used are horse IgG and human thyroglobulin derived from thyroid gland and tetanus toxoid. The antigen is preferably purified prior to use.

The antibody which is the raw material of the F(ab')$_2$ fragment will correspond to the above antigen, and will be human antibody. Among the various human antibodies which may be used, for example IgG antibody, IgM antibody and IgE antibody, IgG antibody is preferred. The antibody can previously have been obtained from human blood by a conventional method, for example the Cohn method, the rivanol precipitation method, the polyethylene glycol method, and the gel filtration method.

F(ab')$_2$ fragment may be prepared by a conventional method, and usually using antibody which has been digested with pepsin. F(ab')$_2$ fragment is preferably separated from the digest and purified.

The separation is preferably carried out by a fractionation technique such as gel filtration, since the molecular weight of the F(ab')$_2$ fragment does not depend on the kind of raw antibody to be used and it is of the order to 100,000.

The complex of the antigen and F(ab')$_2$ fragment is produced by mixing in a solution. The pH of the solution is preferably kept at 5 to 9, and a buffer solution is usually employed. The concentration of the antigen is preferably 1.0 to 10 mg/ml, and that of the F(ab')$_2$ fragment is preferably 1.0 to 10 mg/ml. The preferred molar ratio of the antigen/the F(ab')$_2$ fragment is about 3. The complex formed may be stabilized by NaCl, and accordingly, NaCl is preferably added to the solution. In order to complete formation of the complex, the mixed solution is preferably warmed at 35 to 40°C for 1 to 6 hours.

The complex formed is separated by gel filtration and lyophilised, if necessary. The complex thus obtained is a glycoprotein whose saccharide content determined by the phenol-sulphuric acid method is about 2 to 6%. Its molecular weight determined by the gel filtration method is about 300,000 to 500,000, and its sedimentation coefficient determined by the meniscus depletion method is about 10.8 to 15.2.

When an antibody of this invention is prepared by using the aggregate of F(ab')$_2$ fragment of human immunoglobulin, again any kind of human immunoglobulin may be used although IgG is the most preferred. If desired the immunoglobulin may be purified by any convenient method. F(ab')$_2$ fragment is prepared and purified by the method previously described.

The F(ab')$_2$ fragment is aggregated by heating, acid treatment, treatment with guanidine hydrochloride or treatment with urea. In order to aggregate the F(ab')$_2$ fragment, the F(ab')$_2$ fragment is first dissolved in a saline solution or a buffer red saline solution in a concentration of 1 to 30 mg/ml. When the heating method is used, the solution is heated at 55 to 70°C for 5 to 30 minutes. When acid treatment is carried out, the solution is adjusted to pH 1.5 to 3 by using hydrochloric acid, and allowed to stand for 1 to 8 hours. When the guanidine hydrochloride treatment method is used, guanidine hydrochloride is added to the solution to be present in a concentration of 5 to 8 M the pH of the solution having previously been adjusted to 6.0 to 8.0 by using a buffer solution, and the mixed solution obtained is allowed to stand for from 5 minutes to 18 hours.

The aggregate formed may be purified by gel filtration and lyophilised, if desired.

An antibody embodying this invention is prepared in vivo although when it is a monoclonal antibody it may also be produced in vitro.

When the antibody is produced in vivo, various warm-blooded animals, such as rabbit, guinea pig and chicken may be employed. In order to increase the yield of the antibody, it is preferred that immunotolerance be endowed upon the warm-blooded animal. In the case of a rabbit weighing 3 kg, injection of 10 mg of F(ab')$_2$

fragment leads to appearance of immunotolerance after 1 to 3 days. In the case of other animals, the injection amount may be determined on the basis of the ratio of its body weight/body weight of rabbit (3 kg).

The complex of antigen and F(ab')$_2$ fragment or the aggregate of F(ab')$_2$ fragment is injected into the warm-blooded animal. Injection is carried out several times at intervals of 2 days to 2 weeks. The injection amount each time is preferably, in the case of rabbit, 0.1 to 5 mg, in the case of a guinea pig, 0.05 to 3 mg, and in the case of chicken, 0.1 to 10 mg.

In a procedure for producing the antibody as monoclonal antibody from a warm-blooded animal, 50 to 200 µg of the above complex or the above aggregate may be injected into abdominal cavity of BALB/C strain mouse, and its spleen is isolated after two weeks. A spleen cell is fused with mouse myeloma P3U1 cell for example using polyethylene glycol. The hybridoma thus obtained is cultured and cloned, and a cell capable of producing the antibody of the invention is obtained. This cell is injected into abdominal cavity of a mouse, and multiplied. Then, ascites are collected, and the antibody of the invention is separated from the ascites.

The antibody thus produced is separated from blood by a method of the type used in separating immunoglobulin, for example precipitation using ammonium sulphate, ion-exchange chromatography using DEAE-cellulose, and gel filtration. The Cohn method, the rivanol precipitation method and the polyethylene glycol method are also applicable. The antibody may be further purified by affinity chromatography using Sepharose (Registered Trade Mark) coupled with the same F(ab')$_2$ fragment as that employed in the process to produce this antibody. In this way, antibodies capable of reacting with this F(ab')$_2$ fragment are removed.

On the other hand, when the antibody is separated from ascites, the separation need be carried out only by salting-out with ammonium sulphate followed by gel filtration or affinity chromatography using the carrier coupled with an anti-mouse IgG antibody. There is not need to carry out affinity chromatography using Sepharose coupled with the F(ab')$_2$ fragment.

Since the antibody of the invention specifically reacts with immune complexes and does not react with aggregated IgG, this antibody can be substituted for the anti-antibody employed in the Kano method. The Kano method has not been put in to practical use, because it uses an anti-antibody which is produced in the human body. However, in contrast, the antibody of this invention can be produced by using an animal other then human, and it can also be produced as monoclonal antibody. It is possible to obtain both a complex of an antigen and the F(ab')$_2$ fragment and an aggregate of F(ab')$_2$ fragment of human immunoglobulin readily from waste blood.

The antibody of this invention reacts with a wide range of immune complexes, and these immune complexes can detect by using only one antibody. The present invention makes the detection of immune complex a practical proposition and will assist in the treatment of whatever diseases are caused by the particular immune complex.

The antibody of this invention is not merely an alternative to the anti-antibody of the Kano method. It can also be used for the detection of immune complex by enzyme immunoassay.

The following Examples illustrate this invention:

Example 1

Blood containing anti-horse serum antibody obtained from a healthy person who has been infected with tetanus and given anti-tetanus horse serum was employed. In conventional manner, blood serum was separated from the blood, and purified by salting-out using ammonium sulphate. Then, the precipitate obtained was fractionated by ion exchange chromatography using DEAE cellulose, and the IgG fraction was obtained. This fraction was purified by affinity chromatography using Sepharose 4B (manufactured by Pharmacia AB) coupled with hors IgG, and anti-horse IgG human IgG was obtained.

1000 mg of this human IgG were dissolved in 200 ml of 0.01 M borate buffer solution of pH 4.5. 10 mg of pepsin were added to the solution, and kept at 37°C for 18 hours to produce F(ab')$_2$ fragment. This pepsin digest was separated by gel filtration using Sephadex® G-200 (manufactured by Pharmacia AB). Absorbance of effluent of the Sephadex® G-200 column at 280 nm was measured, and the second peak fractions of the absorbance were collected. The collected fractions which contained the F(ab')$_2$ fragment were then lyophilised.

200 mg of this F(ab')$_2$ fragment and 600 mg of horse IgG were dissolved in 30 ml of 0.001 M Tris-HCl buffer solution of pH 8.0, and the solution was slowly stirred at 37°C for 2 hours. This solution was then applied to a Sephacryl® S-300 (manufactured by Pharmacia AB) column, and the first peak fraction (absorbance at 280 nm) was collected. The first peak fraction was lyophilised to obtain the lyophilised complex of horse IgG and anti-horse IgG human F(ab')$_2$ fragment.

Some of the remaining F(ab')$_2$ fragment pre-viously prepared was in the meanwhile dissolved in a saline solution, and 10 mg of the fragment per one rabbit were injected into 20 rabbits at their ear veins. These 20 rabbits were bled for 2 days and then were admitted as an immunotolerance group.

The above-mentioned lyophilised complex was dissolved in a saline solution in a concentration of 4 mg/ml, and an equal amount of Freund complete adjuvant was added to the solution. The mixture was emulsified and used as antigen. 20 rabbits of the immunotolerance group and 20 rabbits of an untreated group were injected three times each with 0.5 ml of this emulsion every two weeks. A week after each third injection, whole blood was drawn from the carotid of each rabbit.

Blood serum from each rabbit was separated individually, and 12.15 g of ammonium suphate (40% saturation) were added to each 50 ml of the blood serum. The blood serum samples were then stirred for 30 minutes individually, and the precipitate was collected in each case centrifuging at 8000 rpm for 30 minutes. The precipitate was dialysed overnight against 30 l of 0.01 M Tris-HCl buffer solution of pH 8.0 and the dialyzate was applied to the column (2.0 cm×60 cm) of DE-52 (made by (Whatman Separation Ltd.) which had previously been equilibrated with pH 8.0 of 0.02 M Tris-HCl buffer solution. The fractions passing through the column were dialysed overnight against 30 l of 0.01 M borate buffer solution of pH 8.5, and the dialyzate was applied to a column (1.6 cm×10 cm) of Sepharose 4B (manufactured by Pharmacia AB) coupled with horse IgG which had previously been equilibrated with 0.1 M borate buffer solution of pH 8.5. The fraction passing through this column was collected, and this fraction was applied to a column (1.6 cm×10 cm) of Sepharose 4B (manufactured by Pharmacia AB) coupled with anti-horse IgG human F(ab')$_2$ fragment which had previously been equilibrated with 0.1 M borate buffer solution of pH 8.5. The fraction passing through this column was collected, and dialysed overnight against 20 l of saline solution containing 1/1000 M Tris-HCl buffer solution of pH 8.0. The dialyzate was lyophilised, and 10 mg of the lyophilised matter were obtained per rabbit.

The reaction of each lyophilised material with serum from a patient suffering from systemic lupus erythematotsus which gave positive indication with respect to immune complex by the $C_{Iq}$ method was examined by the Ouchterlony method using an agar plate, and when using the lyophilised material as aforesaid of 3 rabbits from the untreated group and of 5 rabbits from the immunotolerance group, a precipitation line was found.

The columns used in this Example was prepared as follows:

8 g of Sepharose 4B which was activated with CNBr were placed on a glass filter, and washed with 1400 ml of 1 mM HCl. Then, the Sepharose was put into a beaker, and 500 mg of horse IgG (or anti-horse IgG human F(ab')$_2$ fragment) which had been dissolved in 100 ml of 0.1 M NaHCO$_3$ buffer solution of pH 9.0 containing 0.5 M NaCl were added to the Sepharose 4B. The mixture was kept at ambient temperature for two hours with occasional stirring, and then filtered. Subsequently, 150 ml of 0.1 M Tris-HCl buffer solution of pH 8.0 were added to this Sepharose 4B, and the mixture was kept at ambient temperature for two hours with occasional stirring, and filtered. The Sepharose 4B coupled with the horse IgG (or the anti-horse IgG human F(ab')$_2$ fragment) on the filter was washed three times with 150 ml of 0.1 M borate buffer solution of pH 8.0, and then twice with 150 ml of 0.1 M acetate buffer solution of pH 4.0. The washed Sepharose 4B was packed in a column, and equilibrated with 0.1 M borate buffer solution of pH 8.5, and used for the purification.

An immune complex test was carried out according to the Kano method using the positive lyophilised material obtained in this Example. For this purpose, the lyophilised material was dissolved in 0.15 M phosphate buffer solution of pH 7.2 containing 0.14 M NaCl in a concentration of 10 µg/ml, and each 20 µl of this solution was mixed with 20 µl of sample serum which had previously been diluted as appropriate. This mixture was kept at 20°C for 60 minutes, and put in a well of a micro titre plate. Then, 20 µl of 1% anti-D antibody sensitized human blood cell belonging to group O Rh positive were added to each well, and kept at 20°C for 30 minutes in a humidified room, and then judged. When a sample serum diluted with 4 times still inhibited the agglutination of the sensitized blood cell, the sample serum was judged to be positive.

The results obtained are tabulated in the following table. In the table, SLE represents the blood serum of a patient subject to systemic lupus erythematosus, and RA represents blood serum of a patient with rheumatoid arthritis. RA had been treated with 0.2 M 2-mercaptoethanol before used in the test. "Addition" means that the sample serum was heated at 50°C for 30 minutes.

| | Number of samples | Percentage of positive | Percentage of positive after addition |
|---|---|---|---|
| SLE | 20 | 40% | 40% |
| RA | 21 | 71% | 71% |
| Healthy Person | 18 | 0% | 0% |

Though heat-aggregated IgG was added to half the number of the sera of healthy persons in a concentration of 200 g per 1 ml of the serum, none indicated positive.

Example 2

F(ab')$_2$ fragment as obtained in Example 1 was dissolved in a saline solution and the solution was injected into the volar veins of 30 guinea pigs in an amount of 5 mg of the fragment per guinea pigs. These 30 guinea pigs wer bled for 3 days, and were then admitted as immunotolerance group. 0.5 ml samples of the same emulsion as that employed in Example 1 were administered four times every two weeks by intracutaneous injection to 30 guinea pigs of the immunotolerance group and 25 guinea pigs of an untreated group. A week after the last injection, whole blood was drawn from the carotid of each guinea pig.

Each 5 ml of blood serum from each guinea pig was separated individually, and treated by the procedure employed in Example 1 to obtain 0.9 mg of lyophilised material per guinea pig.

The reaction of each sample of lyophilised material with the serum of a patient subject to systemic lupus erythematosus which was positive with respect to immune complex was examined in the same manner as in Example 1, and the lyophilised materials from 3 guinea pigs among the untreated group and from 6 guinea pigs among the immunotolerance group were found to be positive.

Example 3

The same $F(ab')_2$ fragment as that used in Example 1 was dissolved in a saline solution, and was injected into the veins of 18 chickens in an amount of 10 mg of fragment per chicken. These 18 chickens were bled for one week, and were admitted as immunotolerance group. The same emulsion as used in Example 1 was administered to each of 18 chickens of an immunotolerance group and 17 chickens of an untreated group of intramuscular injection to the chests three times each week in an amount of 0.5 ml per administration. A week after the last injection, whole blood was drawn from the carotid of each chicken.

Each 70 ml of blood serum of each chicken was separated individually, and treated in the same manner as in Example 1 to obtain 9 mg of lyophilised material per chicken.

The reaction of each sample of lyophilised material with the serum of a patient subject to systemic lupus erythematosus which indicated positive with respect to an immune complex was examined in the same manner as in Example 1, and the lyophilised materials of 2 chickens among the untreated group and of 5 chickens among the immunotolerance group were found to be positive.

Example 4

Blood serum separated from preserved human blood was treated by salting-out using ammonium sulphate, and fractionated by ion exchange chromatography using DEAE cellulose to obtain IgG fraction.

1000 mg of this IgG were dissolved in 200 ml of 0.01 M borate buffer solution of pH 4.3. 10 mg of pepsin were added to this solution, and stirred at 37°C for 18 hours. This pepsin digest solution was separated by gel filtration using Sephadex® G-200 (manufactured by Pharmacia AB). Absorbance of the effluent of the Sephadex G-200 column at 280 nm was measured, and the second peak fractions of the absorbance were collected. The collected fractions were treated with Sepharose 4B (manufactured by Pharmacia AB) coupled with protein A to remove unreacted IgG Fc fragment. The fractions passing through the column were collected, and lyophilised to obtain 500 mg of $F(ab')_2$ fragment.

50 mg of the $F(ab')_2$ fragment were dissolved in a saline solution in a concentration of 10 mg/ml, and heated at 65°C for 5 minutes and then immediately rapidly cooled. The heat treated solution was lyophilised, and the lyophilised matter was used as antigen.

1 ml of the saline solution of unheated $F(ab')_2$ fragment described above was injected into the ear veins of each of 10 rabbits having a body weight of 3 kg. The above-mentioned antigen was dissolved in a saline solution in a concentration of 4 mg/ml. An equal amount of Freund complete adjuvant was added to this solution, and the mixture obtained was emulsified. After 3 days, the emulsion was injected into the 10 rabbits, injection taking place at their front legs and hind legs intracutaneously and at thigh muscle, injection taking place three times every two weeks using 0.5 ml of emulsion per injection. A week after the third injection, whole blood was drawn from the carotids of the animals.

500 ml of blood serum were separated from the blood, and 121 g of ammonium sulphate were added to the serum. The serum was stirred for 30 minutes, and the precipitate formed was collected by centrifuging at 8000 rpm for 30 minutes. This precipitate was dialysed overnight against 100 l of 0.02 M Tris-HCl buffer solution of pH 8.0, and the residue was passed through a column (1.5 cm×80 cm) of DE-52 (made by Whatman Separation Ltd.) which had previously been equilibrated with 0.02 M Tris-HCl buffer solution of pH 8.0. The fractions passing through the column were dialysed over-night against 100 l of 0.01 M borate buffer solution of pH 8.5, and the dialysate was applied to a column (2.0 cm×20 cm) of Sepharose 4B (manufactured by Pharmacia AB) coupled with the unheated $F(ab')_2$ fragment described above which had previously been equilibrated with 0.1 M borate buffer solution of pH 8.5. The fractions passing through the column were collected, and dialysed overnight against a saline solution containing 1/1000 M Tris-HCl buffer solution of pH 8.0. The dialysate was lyophilised, and 500 mg of the lyophilised antibody material of the invention were obtained.

An immune complex test was carried out according to the ELISA method using the lyophilised antibody material. The lyophilised antibody material was dissolved in 0.02 M phosphate buffer solution of pH 0.02 M in a concentration of 60 µg/ml, and 60 µl portions of the solution were placed in each well of a plate for the ELISA test having 96 wells. Then, the solution in the wells was allowed to stand at 4°C overnight, and the antibody in the solution was adsorbed on the surface of the wells. Each well was washed, and 60 µl of 0.02 M phosphate buffer solution containing 1% bovine serum albumin were placed in each well. 10 µl of a human blood serum diluted 5 times with a saline solution were added to each well and reacted at room temperature for 2 hours. After the reaction, the solution in each well was drawn off, and the wells were washed. 50 µl of peroxidase conjugated anti-human IgG goat antibody were placed in each well, and reaction for 2 hours was allowed to take place. After washing, 50 µl of peroxidase substrate of $H_2O_2/2,2'$ - azino - di - (3 - ethyl - benzo-thiazoline - 6 - sulphonic acid) were added to each well, and reacted for 30 minutes at room temperature. After the reaction, the absorbances of the reaction mixtures at 405 nm were measured.

As a result, with the sera of healthy persons, $OD_{405}=0.02$ (n=20), with the sera of patients subject to systemic lupus erythematosus, $OD_{405}=1.8—0.5$ (n=40), and with patients subject to chronic rheumatoid arthritis, $OD_{405}+2.0—1.2$ (n=21). When 100 µg of heat-aggregated human IgG were added per 1 ml of the serum of the healthy person, the $OD_{405}$ value of the serum was also 0.02.

Example 5

Antigen obtained in the same manner as in Example 4 was dissolved in a saline solution in a concentration of 1000 µg/ml, and 0.1 of this solution was injected into abdominal cavity of BALB/C mouse of 8 weeks growth. After one week, the injection was repeated again in the same manner. At the end of each of two following weeks 50 µg/0.1 ml of a saline solution of the antigen were injected into the tail vein of the mouse and 3 days later, its spleen was isolated. This spleen was ground, and spleen cells were separated. The spleen cells were fused with mouse myeloma P3UI cells using polyethylene glycol 1500. A hybridoma thus produced was put in each well of a plate having 96 wells, and cultured in HAT medium. The cell of each well was examined by the ELISA method, the cells of 6 wells being found positive. These positive cell values means that the cells in question react with an aggregated human $F(ab')_2$ fragment and do not react with a human $F(ab')_2$ fragment which is not aggregated. These positive cells were diluted by the limit dilution method, and cloned, and 7 positive cell lines were obtained.

Each positive cell line was multiplied in 10% FCS—RPMI medium, $10^7$ of the multiplied cells being injected into the abdominal cavities of BALB/C mice (previously injected Pristance® (Aldrich) 0.5 ml). After two weeks, about 10 ml of ascites were withdrawn. This ascites was treated by the precipitation method using ammonium sulphate solution of 45% by weight saturation, and dialysed against 10 l of 0.02 M tris-HCl buffer solution of pH 8.0. The dialysate was applied to a Sephadex® G-200 (manufactured by Pharmacia AB) column which had previously been equilibrated with 0.1 M phosphate buffer solution of pH 7.0, and the fractions corresponding to molecular weights of 150,000 to 200,000 were collected. The absorbance of the collected fractions at 280 nm was measured, and it was determined that these fractions contained 5 to 12 mg of IgG.

These fractions were reacted with the immune complex separated from a serum of a SLE patient by means of the ELISA method, and the fractions obtained with 3 cell lines were reacted.

When the immune complex test was carried out according to the ELISA method, using these reactive fractions and the sera of healthy persons, SLE patients and RA patients, the same absorbances as in Example 4 were obtained.

Example 6

Human $F(ab')_2$ fragment prepared in the manner described in Example 4 was dissolved in a saline solution in a concentration of 10 mg/ml, and the pH of the solution was adjusted to 2.0 by using 0.1 N HCl. This solution was allowed to stand at 4°C for 18 hours.

The acid treated solution was neutralized with 0.1 N NaOH, and an equal amount of Freund complete adjuvant was added to the solution. The mixture was emulsified, and the emulsion was administered 4 times by intracutaneous injection to the backs of 30 guinea pigs which had previously been given immunotolerance every two weeks, injection being carried out using 0.5 ml (containing 0.1 mg of the antigen) of this emulsion for each injection of one guinea pig. One week after the last injection, whole blood was drawn from the carotids of the guinea pigs.

When this blood was treated in the same manner as in Example 4, 40 mg of lyophilised anti-immune complex antibody material were obtained.

Example 7

Human $F(ab')_2$ fragment prepared in the manner described in Example 4 was dissolved in 0.02 M phosphate buffer solution of pH 8.0 in a concentration of 10 mg/ml, and guanidine. HCl was added to the solution in a concentration of 8 M. This solution was allowed to stand at ambient temperature for 3 hours, and dialysed against a saline solution.

The residue was mixed with an equal volume of Freund complete adjuvant, and emulsified. The emulsion was administered 3 times by intramuscular injection to the chests of 18 chickens which had previously been given immunotolerance every two weeks, 0.5 ml of emulsion being administered to a chicken each time. One week after the last injection whole blood was drawn from the carotids of the chickens.

The blood was treated in the same manner as Example 4, and 160 mg of lyophilised anti-immune complex antibody material were obtained.

**Claims**

1. An anti-immune complex antibody which is a glycoprotein having a molecular weight of from 150,000 to 180,000 when determined by the gel filtration method, which antibody reacts with an immune complex in blood serum of a patient subject to systemic lupus erythematosus and with an immune complex in a blood serum of a patient subject to rhematoid arthritis, but does not react with aggregated IgG, has a sedimentation coefficient of from 6.5 to 7.1 determined by the Meniscus depletion method, and has a saccharide content of from 2% to 4.5% by weight as determined by the Phenol-Sulphuric acid method and has an isoelectric point of from 5.8 to 8.7.

2. A method of producing an anti-immune complex antibody according to claim 1, which comprises bringing into contact with each other in a solution medium an antigen and the $F(ab')_2$ fragment of a human antibody against this antigen to produce a complex of the antigen and the $F(ab')_2$ fragment, raising the anti-immune complex antibody by using the complex as antigen and recovering the antibody from the warm blooded animal in which it has been produced.

3. A method of producing an anti-immune complex antibody according to claim 1, which comprises raising the anti-immune complex antibody by using an aggregate of $F(ab')_2$ fragment of human immunoglobulin as antigen and recovering the antibody from the warm blooded animal in which it has been produced.

4. A method as claimed in Claim 2, wherein the antigen and the $F(ab')_2$ fragment are brought together in an aqueous medium.

5. A method as claimed in Claim 4, wherein the antigen is horse IgG, human thyroglobulin derived from thyroid gland or tetanus toxoid.

6. A method as claimed in any one of Claims 2, 4 and 5, wherein the human antibody is IgG antibody.

7. A method as claimed in any one of Claims 2 and 4 to 6, wherein the complex is produced by mixing in a solution at a pH of 5 to 9 employing an antigen concentration of from 1.0 to 10 mg/ml and a $F(ab')_2$ fragment concentration of from 1.0 to 10 mg/ml.

8. A method as claimed in Claim 3, wherein the $F(ab')_2$ fragment is aggregated by heating, acid treatment, treatment with guanidine hydrochloride or treatment with urea, the $F(ab')_2$ fragment being employed in saline solution in a concentration of from 1 to 30 mg/ml.

9. A method as claimed in Claim 8, wherein the solution is heated at 55 to 70°C for 5 to 30 minutes in the heating, adjusted to pH 1.5 to 3 using hydrochloric acid and allowed to stand for from 1 to 48 hours in the acid treatment, and, in the guanidine hydrochloride treatment has guanidine hydrochloride added to it for the guanidine hydrochloride to be present in a concentration of 5 to 8M, the pH of the solution then having been previously adjusted to 6.0 to 8.0 and then being allowed to stand for from 5 minutes to 18 hours.

10. A method as claimed in any one of Claims 2 to 9, wherein the anti-immune complex antibody is raised in vivo in a warm blooded animal which has preferably had immunotolerance endowed upon it.

11. A method of producing an anti-immune complex antibody according to claim 1, which is prepared as a monoclonal antibody from a hybridoma derived from a warm-blooded animal to which either the complex of claims 2 and 4—7 or the aggregate of claims 3, 8 and 9 has been injected.

**Patentansprüche**

1. Anti-Immunkomplex-Antikörper, welcher ein Glykoprotein mit einem Molekulargewicht von 150.000 bis 180.000, bestimmt nach der Gel-Filtrationsmethode, ist, mit einem Immunkomplex im Blutserum eines Patienten mit generalisierter Erythematodes und mit einem Immunkomplex im Blutserum eines Patienten mit primär chronischem Glenkrheumatismus, jedoch nicht mit aggregiertem IgG reagiert, einen Sedimentations-Koeffizienten von 6,5 bis 7,1, bestimmt nach der "Meniscus depletion"—Methode, hat, einen Saccharidgehalt von 2 bis 4,5 Gew.-%, bestimmt nach der Phenol-Schwefelsäuremethode, aufweist und einen isoelektrischen Punkt zwischen 5,8 und 8,7 hat.

2. Verfahren zur Herstellung des Anti-Immunkomplex-Antikörpers nach Anspruch 1, welches umfaßt: das in Berührung bringen eines Antigens und eines $F(ab')_2$-Fragments eines menschlichen Antikörpers gegen dieses Antigen in einem Lösungsmittelmedium zwecks Bildung eines Komplexes aus dem Antigen und dem $F(ab')_2$-Fragment, die Vervielfältigung des Anti-Immunkomplex-Antikörpers unter Verwendung des Komplexes als Antigen und Wiedergewinnung des Antikörpers aus dem Warmblüter, in dem er gebildet worden ist.

3. Verfahren zur Herstellung des Anti-Immunkomplex-Antikörpers nach Anspruch 1, welches umfaßt: die Vervielfältigung des Anti-Immunkomplex-Antikörpers unter Verwendung eines Aggregats von dem $F(ab')_2$-Fragment eines menschlichen Immunglobulins als Antigen und Wiedergewinnung des Antikörpers aus dem Warmblüter, in dem es gebildet worden ist.

4. Verfahren nach Anspruch 2, wobei das Antigen und das $F(ab')_2$-Fragment in einem wässerigen Medium zusammengebracht werden.

5. Verfahren nach Anspruch 4, wobei das Antigen Pferde-IgG, menschliches Thyreoglobulin aus der Schilddrüse oder Tetanustoxoid ist.

6. Verfahren nach einem jeden der Ansprüche 2, 4 und 5, wobei es sich bei dem menschlichen Antikörper um IgG-Antikörper handelt.

7. Verfahren nach einem jeden der Ansprüche 2 und 4 bis 6, wobei der Komplex gebildet wird durch Mischen in einer Lösung bei einem pH von 5 bis 9 unter Verwendung einer Antigen-Konzentration von 1,0 bis 10,0 mg/ml und einer $F(ab')_2$-Fragment-Konzentration von 1,0 bis 10,0 mg/ml.

8. Verfahren nach Anspruch 3, wobei das $F(ab')_2$-Fragment aggregiert wird durch Erhitzen, durch Säurebehandlung, Behandlung mit Guanidinhydrochlorid oder Harnstoff und das F(ab')$_2$-Fragment in einer Konzentration von 1 bis 30 mg/ml in einer Salzlösung vorliegt.

9. Verfahren nach Anspruch 8, wobei die Lösung im Falle der Hitzebehandlung auf 55 bis 70°C erhitzt und während 5 bis 30 Minuten dabei gehalten wird, im falle der Säurebehandlung der pH mittels HCl auf 1,5 bis 3 eingestellt und während 1 bid 48 Stunden belassen wird sowie im Falle der Guanidinhydrochlorid-Behandlung dieses bis zu einer Konzentration von von 5 bis 8 M nach vorheriger Einstellung vom pH der Lösung auf 6 bis 8 zugegeben wird und dann eine Stehenlassen während 5 Minuten bis 18 Stunden erfolgt.

10. Verfahren nach einem jeden der Ansprüche 2 bis 9, wobei der Anti-Immunkomplex-Antikörper in vivo in einem Warmblüter, der vorzugsweise immuntolerant gemacht worden ist, vervielfältigt wird.

11. Verfahren zur Herstellung des Anti-Immunokomplex-Antikörpers nach Anspruch 1, welcher als monoklonaler Antikörper aus einem Hybridom eines Warmblüters, dem entweder der Komplex der Ansprüche 2 und 4 bis 7 oder das Aggregat der Ansprüche 3, 8 und 9 injiziert worden ist gebildet wird.

**Revendications**

1. Anticorps anti-immuno-complexe, qui est une glycoprotéine ayant un poids moléculaire allant de 150 000 à 180 000 tel qu'on le mesure par la méthode de filtration sur gel, lequel anticorps réagit avec un immuno-complexe dans le sérum sanguin d'un malade sujet à un lupus erythematosus systémique, et avec un immuno-complexe dans le sérum sanguin d'un malade sujet à une arthrite rhumatoîde, mais ne réagit pas avec les IgG agrégés, possède un coefficient de sédimentation allant de 6,5 à 7,1, mesuré par la méthode de déplétion du ménisque, possède une teneur en saccharide allant de 2 à 4,5% en poids, tel que déterminé par la méthode au phénol-acide sulfurique, et possède un point isoélectrique allant de 5,8 à 8,7.

2. Procédé de production d'un anticorps anti-immuno-complexe conforme à la revendication 1, qui comprend la mise en contact l'un avec l'autre dans un milieu formant solution d'un antigène et du fragment F(ab')$_2$ d'un anticorps humain contre cet antigène, afin de produire un complexe de l'antigène et du fragment F(ab')$_2$, l'augmentation du taux d'anticorps anti-immuno-complexe par l'emploi du complexe comme antigène, et la récupération de l'anticorps à partir de l'animal à sang chaud dans lequel il a été produit.

3. Procédé de production d'un anticorps anti-immuno-complexe conforme à la revendication 1, qui comprend l'augmentation du taux d'anticorps anti-immuno-complexe par l'emploi d'un agrégat du fragment F(ab')$_2$ de l'immunoglobuline humaine comme antigène, et la récupération de l'anticorps à partir de l'animal à sang chaud dans lequel il a été produit.

4. Procédé tel que revendiqué dans la revendication 2, dans lequel l'antigène et le fragment F(ab')$_2$ sont placés ensemble dans un milieu aqueux.

5. Procédé tel que revendiqué dans la revendication 4, dans lequel l'antigène la IgG de cheval, est la thyroglobuline humaine dérivée de la glande thyroîde, ou l'anatoxine du tétanos.

6. Procédé tel que revendiqué dans l'une quelconque des revendication 2, 4, et 5, dans lequel l'anticorps humain est l'anticorps IgG.

7. Procédé tel que revendiqué dans l'une quelconque des revendications 2 et 4 à 6, dans lequel on produit le complexe par mélange dans une solution à un pH de 5 à 9, en employant une concentration d'antigène de 1,0 à 10 mg/ml et une concentration de fragment F(ab')$_2$ de 1,0 à 10 mg/ml.

8. Procédé tel que revendiqué dans la revendication 3, dans lequel le fragment F(ab')$_2$ est agrégé par chauffage, par traitement à l'acide, par traitement au chlorhydrate de guanidine, ou par traitement à l'urée, le fragment F(ab')$_2$ étant employé en solution saline à une concentration de 1 à 30 mg/ml.

9. Procédé tel que revendiqué dans la revendication 8, dans lequel on chuaffe la solution à 55—70°C pendant 5 à 30 minutes au cours du chauffage, on ajuste son pH à 1,5—3 en employant de l'acide chlorhydrique, et on la laisse reposer pendant 1 à 48 heures, ceci dans le traitement à l'acide, et, dans le traitement au chlorhydrate du guanidine, on y ajoute du chlorhydrate de guanidine en quantité telle que celui-ci soit présent en une concentration de 5 à 8 M, le pH de la solution ayant alors été préalablement ajusté à 6,0—8,0, et on la laisse reposer pendant de 5 minutes à 18 heures.

10. Procédé tel que revendiqué dans l'une quelconque des revendications 2 à 9, dans lequel l'anticorps anti-immuno-complexe est produit in vivo dans un animal à sang chaud que a de préférence été doté d'immunotolérance.

11. Procédé de production d'un anticorps anti-immuno-complexe conforme à la revendication 1, qui est préparé comme un anticorps monoclonal à partir d'un hybridome dérivé d'un animal à sang chaud auquel soit le complexe des revendications 2 et 4—7, soit l'agrégat des revendications 3, 8 et 9, a été injecté.